(19) Europäisches Patentamt
European Patent Office
Office européen des brevets

(11) EP 0 583 651 B1

(12) EUROPÄISCHE PATENTSCHRIFT

(45) Veröffentlichungstag und Bekanntmachung des Hinweises auf die Patenterteilung:
**18.06.1997 Patentblatt 1997/25**

(51) Int Cl.6: **C07C 69/96**, A23L 1/226, A24B 15/34

(21) Anmeldenummer: **93111919.2**

(22) Anmeldetag: **26.07.1993**

(54) **Verfahren zur Erzeugung eines physiologischen Kühleffekts und dafür geeignete wirksame Verbindungen**

Process to generate a physiological cooling effect and suitable compounds for this process

Procédé pour causer une impression de fraicheur physiologique et composés actifs utilisables dans ce procédé

(84) Benannte Vertragsstaaten:
**AT BE CH DE DK ES FR GB GR IE IT LI LU NL PT SE**

(30) Priorität: **06.08.1992 DE 4226043**

(43) Veröffentlichungstag der Anmeldung:
**23.02.1994 Patentblatt 1994/08**

(73) Patentinhaber: **HAARMANN & REIMER GMBH**
**D-37601 Holzminden (DE)**

(72) Erfinder:
- **Dr. Pelzer, Ralf**
**D-37699 Fürstenberg (DE)**
- **Dr. Surburg, Horst**
**D-37603 Holzminden (DE)**
- **Dr. Hopp, Rudolf**
**D-37603 Holzminden (DE)**

(74) Vertreter: **Zobel, Manfred, Dr. et al**
**BAYER AG**
**Konzernbereich RP**
**Patente und Lizenzen**
**51368 Leverkusen (DE)**

(56) Entgegenhaltungen:
**US-A- 3 419 543**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 583 651 B1

Printed by Jouve, 75001 PARIS (FR)

## Beschreibung

Die Erfindung betrifft ein Verfahren zur Erzeugung eines physiologischen Kühleffekts auf der Haut oder Schleimhäute durch Aufbringen eines mittels ohne störenden Eigengeruch und Eigengeschmack, Offenbar kommt die Wirkung, die der betroffenen Person den Eindruck der Frische vermittelt, durch Stimulierung der entsprechenden Rezeptoren des menschlischen Nervensystems zustande. Weiterhin betrifft die Erfindung neue Verbindungen, die diese Wirkung hervorrufen können.

Die bekannteste natürliche Verbindung mit physiologischer Kühlwirkung ist zweifellos das im Pfefferminzöl (ex Mentha arvensis) vorkommende (-)-Menthol. Es wird beispielsweise für die Herstellung von Zahnreinigungsmitteln, Mundwässern, Nahrungsmitteln, Getränken und Kosmetika eingesetzt. Jedoch werden das typische intensive Pfefferminz-Eigenaroma und der bitter-brennende Eigengeschmack oft als störend empfunden.

Der in der DE-OS 2 433 165 vorgeschlagenen N-Acetylglycin-menthylester sowie die in der DE-OS 2 339 661 vorgeschlagenen Mentholester heterocyclischer Carbonsäuren sind bitter, und die in der US-PS 3 830 930 vorgeschlagenen Menthylketoester sind z.T. anhaltend bitter und weisen nur eine schwache Kühlwirkung auf.

Aus der DE-OS 2 608 226 sind Mentholester natürlich vorkommender $C_2$-$C_6$-Hydroxycarbonsäuren (die gegebenenfalls ihrerseits an der Hydroxygruppe mit $C_1$-$C_4$-Carbonsäuren verestert sind) bekannt, die geruch- und schmacklos sind und eine langanhaltende Kühlwirkung aufweisen. Insbesondere das 1-Menthyllactat hat sich in der Praxis bewährt. Allerdings ist das Produkt nicht basenstabil, so daß es nicht für alle Anwendungen (z.B. Seifen) geeignet ist.

Auch andere Produkte werden bereits in der Praxis eingesetzt, beispielsweise 3-1-Menthoxypropan-1.2-diol (EP-PS 80 148) und N-Ethyl-p-menthan-3-carboxamid (DE-OS 2 205 255 und 2 413 639).

In der DE-OS 2 022 369 wird 1-Menthanylethylcarbonat als Kühlmittel vorgeschlagen. Diese Substanz weist einen deutlichen fruchtig-orangenartigen, etwas holzigen Eigengeruch auf. Die Kühlwirkung ist im Vergleich zu 1-Menthol sowohl in reiner Form, vor allen Dingen aber in Medien wie zum Beispiel Fondantmasse ganz erheblich schwächer. Es besteht jedoch ein stark zunehmender Bedarf nach Mitteln, die bei fehlendem Eigengeruch und Eigengeschmack eine Kühlwirkung besitzen, die an Intensität möglichst das Menthol erreicht oder übertrifft.

Überraschenderweise wurde nun gefunden, daß gemischte Kôhlensäurederivate, die freie polare Gruppierungen besitzen, im Gegensatz zu solchen ohne diese freien polaren Gruppierungen einen Kühleffekt besitzen, der teilweise höher als der des Menthols ist, und die gleichzeitig weitgehend geschmacks- und geruchsneutral sind.

Gegenstand der Erfindung ist ein Verfahren zur Erzeugung eines physiologischen Kühleffekts auf der Haut oder Schleimhäute durch Aufbringen mindestens einer Verbindung der Formel

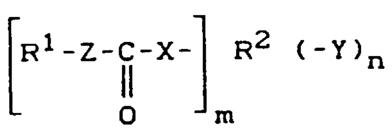

(I)

worin

- $R^1$ $C_4$-$C_{20}$-Alkyl, $C_5$-$C_{20}$-Cycloalkyl oder -Heterocycloalkyl oder $C_5$-$C_{20}$-Alkoxy, $C_6$-$C_{12}$-Aryl, $C_5$-$C_{10}$-Heteroaryl oder $C_7$-$C_{11}$-Aralkyl,
- $R^2$ einen m + w'n-wertigen aliphatischen $C_1$-$C_8$-Rest, cycloaliphatischen oder heterocycloaliphatischen $C_3$-$C_{15}$-Rest, araliphatischen $C_7$-$C_{20}$-Rest, einen Alkoxy- oder Acyloxy-haltigen aliphatischen $C_3$-$C_{15}$-Rest,
- Z und X unabhängig voneinander -O-, -S- oder -NH-,
- Y Hydroxy, $C_1$-$C_{10}$-Alkoxy, $C_2$-$C_6$-Acyloxy, Amino, Mercapto oder -O-$R^3$-O-,
- $R^3$ $C_1$-$C_6$-Alkylen,
- w die Wertigkeit des Restes Y, vorzugsweise 1 oder 2, und
- m und n unabhängig voneinander ganze Zahlen von 1 bis 8 bedeuten mit der Maßgabe, daß die Summe m + n maximal 12 ist.

Bevorzugte Reste $R^1$ umfassen $C_1$-$C_3$-substituiertes Cyclohexyl, vorzugsweise Menthan-3-yl, und verzweigte $C_3$-$C_8$-Alkylreste.

Bevorzugte Reste $R^2$ umfassen beispielsweise Ethylen, Propylen, $CH_3$-$C(CH_2$-$)_3$, $CH_3CH_2C(CH_2$-$)_3$, $C(CH_2$-$)_4$ und dreiwertige $C_6$-Kohlenwasserstoffreste.

Bevorzugte Verbindungen I umfassen insbesondere die Verbindungen

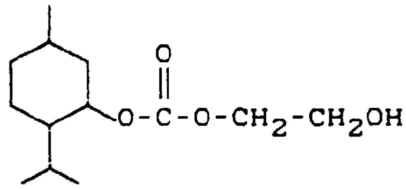

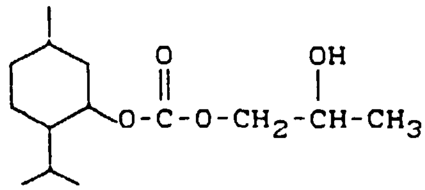

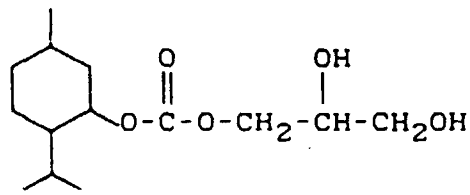

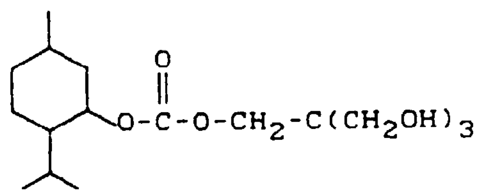

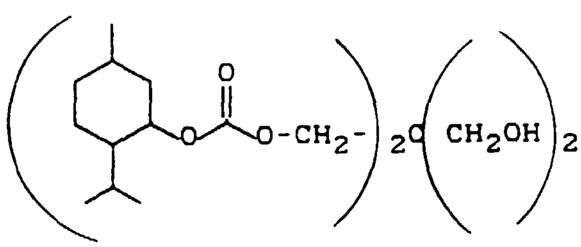

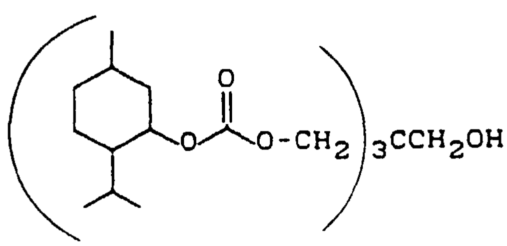

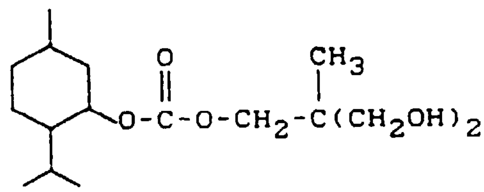

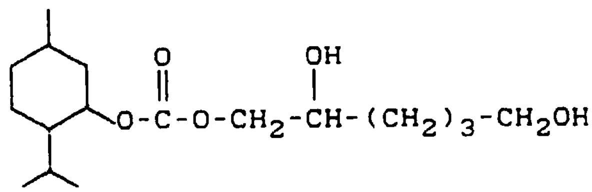

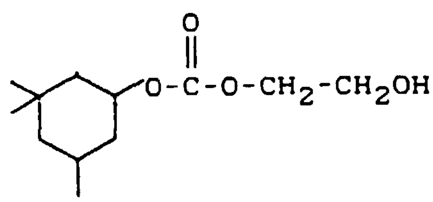

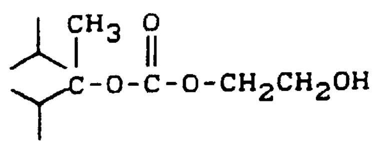

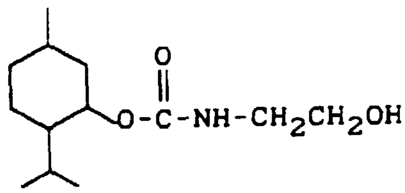

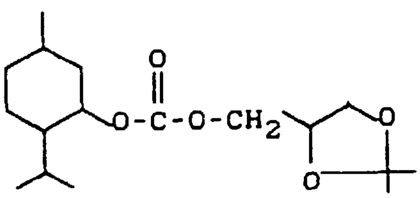

Aus der US-PS 3 419 543 sind Verbindungen der Formel

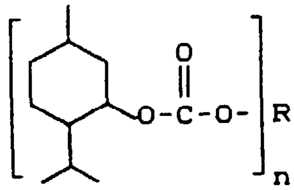

bekannt,
worin n von der Art des Restes R abhängt und R den Rest eines Mono-, Di-, Tri- oder Polysaccharids oder eines Glykols (erhalten durch Abstraktion mindestens einer Hydroxylgruppe) bedeutet. Die Verbindungen werden als Tabakzusätze empfohlen und sollen sich in der Tabakglut zersetzen, so daß Menthol frei wird. Eine physiologische Kühlwirkung der unzersetzten Verbindungen selbst wird weder offenbart noch nahegelegt.

Die übrigen Verbindungen der Formel I sind neu, Weiterer Gegenstand der Erfindung sind also die Verbindungen I mit der Maßgabe, daß nicht gleichzeitig

| | |
|---|---|
| $R^1$ | Menthyl, |
| Z | Sauerstoff, |
| X | Sauerstoff, |
| $R^2(Y)_n$ | den Rest einer Polyhydroxylverbindung aus der Reihe Monosaccharide, Disaccharide, Trisaccharide, Polysaccharide, Glykole bedeuten. |

Die Verbindungen I können durch basisch-gesteuerte Umsetzung von Chlorameisensäureestern mit den entsprechenden Alkoholen, Aminen oder Thiolen oder durch gestaffelte Reaktion von Phosgen, Diphosgen, Triphosgen oder vergleichbaren aktiven Kohlensäurederivaten mit einem $R^1$-Alkohol, -Amin oder-Thiol einerseits und den entsprechenden $R^2$-Alkoholen, -Aminen oder -Thiolen andererseits hergestellt werden. Man wird vorzugsweise in äquimolaren Verhältnissen, gegebenenfalls aber auch mit einer Komponente im Überschuß arbeiten. Geeignete Basen zur Reaktion sind organische Amine wie Pyridin oder Trialkylamine oder anoroanische Basen wie NaOH, KOH, $Na_2CO_3$.

Die Verbindungen (I) weisen teilweise asymmetrische C-Atome auf; bei ihnen kann daher optische Isomerie auftreten. Je nach Ausgangsmaterial und den angewendeten Herstellungsmethoden können sie als Gemische der optischen Isomeren oder als reine Isomere vorliegen. Die Kühlwirkung der Isomeren kann unterschiedlich sein, so daß das eine oder andere Isomere bevorzugt sein kann.

Die Verbindungen I werden bevorzugt auch in Kombination mit Menthol und/oder anderen bekannten Kühlsubstanzen, wie sie z.B. in den auf Seite 2 genannten Patentschriften aufgezeigt sind, eingesetzt, so daß deren teilweise schwache Kühlwirkung deutlich verlängert und/oder verstärkt wird. Sinnvoll ist auch eine Kombination natürlicher, naturidentischer oder synthetischer Aromakomponenten, insbesondere des Mint-Tpys, mit den Verbindungen I, um deren Kühlwirkung und Frischeeindruck zu verstärken.

Die Kühlmittel können neben den Verbindungen I Träger und/oder Verdünnungsmittel enthalten.

Deren Art richtet sich nach dem vorgesehenen Zweck des Mittels.

Das erfindungsgemäße Verfahren kann überall da angewendet werden, wo eine physiologische Kühlwirkung erwünscht ist. Zusammensetzungen, in denen die Kühlmittel gern verwendet werden, sind beispielsweise

<u>Nahrungsmittel</u>

- Backwaren, Konfekt, Süßwaren (Bonbons, Schokoladen), Waffelfettmasse,
- alkoholische Getränke (Biere, Schnäpse, Liköre),
- nicht alkoholische Getränke (Fruchtsäfte, Limonaden, Cola, Milchgetränke, Mineralwasser, in Wasser auflösbare Tabletten),
- Milchprodukte, Joghurts,
- Eisprodukte,
- Kaugummi,
- Geleeprodukte, Marmeladen, Konfitüren,
- Puddings.

Kosmetische Mittel

- Rasiercreme, -schaum, -gel und -seife,
- After Shave Produkte mit und ohne Alkohol: Lotion, Milch, Creme, Gel, Balm,
- Pre Shave-Produkte: Lotion, Milch, Creme, Gel, Balm,
- Hautpflegeprodukte: Creme, Lotion, Milch, Gel, Schaum, Öl,
- Hautreinigungsprodukte: Creme, Lotion, Tonic, Tuch, Pad,
- Hauterfrischungsprodukte: Gel, Lotion, Erfrischungstuch,
- Parfüms: EdC, EdT, EdP, Extrait, Splash Cologne
- Deodorant- und Antiperspirantprodukte: Stick, Roll-on, Spray, Creme, Puder,
- After Sun-Produkte: Lotion, Balm, Milch, Gel, Creme, Spray, Duschbad, Shampoo,
- Lippenpflegeprodukte: Stick, Pomade,
- Enthaarungsprodukte: Creme, Milch, Schaum,
- Gesichtsmasken,
- Sportive Produkte: Öl, Tonic, Milch, Creme, Gel, Spray, Balm,
- Haareinigungs-/-pflegeprodukte: Shampoo, Spülung, Kur, Tonic, Pomade, Öl, Gel, Creme, Balm, Haarfarben, Dauer wellen,
- Seifen: Toilettenseife, Flüssigseife
- Badeprodukte: Öl, Schaumbad,
- Körperpuder,
- Fußpflegeprodukte: Bad, Creme, Tonic, Gel, Milch, Spray,
- Mund- und Zahnpflegeprodukte: Zahncreme, Zahngel, Mundwasser, Mundspray, Gurgelpräparate, Zahnstocher, Zahnseide, Gebißreinigungsmittel, Mundduschе, Kaugummi.

Tabakwaren

- Zigaretten, Zigarren,
- Kautabak, Schnupftabak, Pfeifentabak, Filtertips.

Haushaltsprodukte

- Hand-Geschirrspülmittel, Wäscheweichspülmittel, Wäsche-Imprägnierungsmittel, Toilettenpapier, Air Freshener, Trinkstrohhalm.

Pharmazeutische Präparate

Antiseptische Salben, säurebindende Magenmittel, Linimente, orale Analgetika, Hustensaft, Rachenpastillen, zahnmedizinische Spülungen.

Textilbehandlungsmittel

- Waschmittel, Wäscheweichspüler, Appreturen

Die Endprodukte enthalten die Verbindungen I in einer Menge, die ausreicht, um das erwünschte Kälteempfinden hervorzurufen. In der Regel kommen 0,01 bis 3, vorzugsweise 0,05 bis 1, Gewichtsprozent, bezogen auf das Gewicht der Gesamtzusammensetzung, zur Anwendung.

Die folgenden Beispiele erläutern die Erfindung. Bei den Prozentangaben handelt es sich, sofern nichts anderes angegeben ist, um Gewichtsprozente.

Beispiele

Beispiel 1

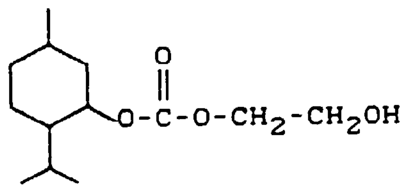

In einem 1000 ml-Dreihalskolben werden 62 g (1 mol) Ethylenglycol und 48 g (0,6 mol) Pyridin vorgelegt. Innerhalb von 2 Stunden werden 109 g (0,5 mol) Chlorameisensäurementhylester zudosiert. Die Reaktion ist deutlich exotherm und erreicht zwischenzeitlich knapp 50°C. Das Gemisch wird 2 Stunden bei 60°C nachgerührt. Mit konz. Salzsäure wird auf pH = 2 angesäuert (ca. 70 g HCl), wobei die Reaktionstemperatur durch Kühlung bei ca. 30°C gehalten wird. Nach Phasentrennung wird die wäßrige Phase mit 50 ml Ether extrahiert, und die vereinigten organischen Phasen werden eingeengt. Der Rückstand wird mit 300 ml Hexan versetzt, wobei das Produkt zum größten Teil auskristallisiert (52 g). Die verbleibende Mutterlauge wird destilliert und ergibt bei einem Siedepunkt von 137-143°C (0,2 mbar) 27 g Destillat, das durch Waschen mit Hexan zur Kristallisation gebracht wird.

Das gereinigte Kristallisat hat einen Schmp. von 51,4 bis 51,5°C und einen Drehwert $\alpha_D^{20}$ = -67,2° (10 %ig in Ethanol).

Beispiel 2

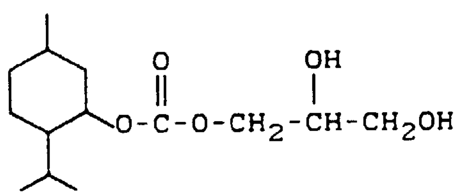

Verfahren analog Beispiel 1 aus 54,5 g (0,25 mol) ameisensaurementhylester und 92,1 g (1 mol) Glycerin. Das Reaktionsgemisch besteht zu 91,3 % aus der gewünschten Verbindung und besitzt folgende Eigenschaften: $D_{25}^{25}$ = 1,0750; $n_D^{20}$ = 1,4720; $\alpha_D^{25}$ = -59,60° (10 %ig in Ethanol). Ausbeute: 73,4 % d.Theorie, bezogen auf Chlorameisensäurementhylester.

Beispiel 3

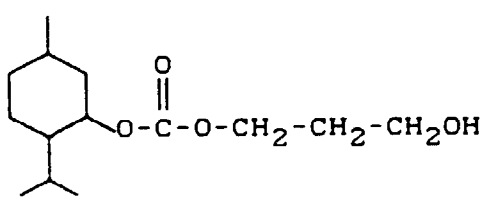

Verfahren analog Beispiel 1 aus 109 g (0,5 mol) Chlorameisensäurementhylester und 76,1 g (1 mol) Propandiol-1,3; Isolierung durch Destillation. $D_{25}^{25}$ = 1,0218; n = 1,4615: $\alpha_D^{25}$ -64,8° (10 %ig in Ethanol); Ausbeute: 63,1 % d.Theorie, bezogen auf Chlorameisensäurementhylester.

Beispiel 4

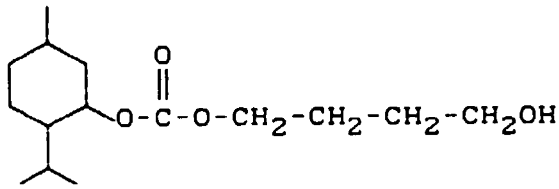

Verfahren analog Beispiel 1 aus 100 g (0,46 mol) Chlorameisensaurementhylester und 49,6 g (0,55 mol) Butandiol-1,4; Isolierung durch Destillation.
$D_{25}^{25}$= 1,0111; $n_D^{20}$ = 1,4621; $\alpha_D^{25}$ = -58,5° (10 %ig in Ethanol); Ausbeute: 51,4 % d.Theorie, bezogen auf Chlorameisensäurementhylester.

Beispiel 5

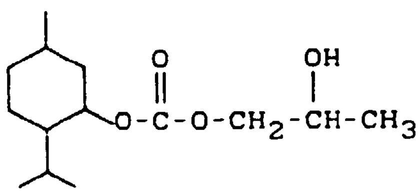

Verfahren analog Beispiel 1 aus 100 g (0,46 mol) Chlorameisensäurementhylester und 42 g (0,55 mol) Propandiol-1,2; Reinigung durch Destillation.

Das Destillat (130 g) besteht zu 89,1 % aus der gewünschten Verbindung und besitzt folgende Eigenschaften: $D_{25}^{25}$ = 1,0154; $n_D^{20}$ = 1,4578; $\alpha_D^{25}$ = -62,2° (10 %ig in Ethanol); Ausbeute: 82,1 % d.Theorie, bezogen auf Chlorameisensäurementhylester.

Beispiel 6

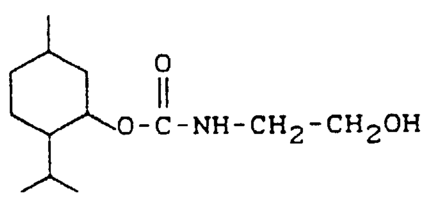

In einem 1000 ml-Dreihalskolben werden 92 g (1,5 mol) Ethanolamin in 200 ml Methyl-tert.-butylether (MTBE) vorgelegt. Bei anfänglich Raumtemperatur werden ohne Kühlung 109 g (0,5 mol) Chlorameisensäurementhylester zudosiert, wobei deutliche Temperaturerhöhung eintritt und er wird 3 Stunden bei 55°C nachgerührt.

Zu dem zweiphasigen, kristallin-breiigen Reaktionsgemisch werden 100 ml Wasser zugegeben, die Phasen getrennt, die wäßrige Phase zweimal mit MTBE (je 100 ml) extrahiert und die vereinigten MTBE-Phasen am Rotationsverdampfer eingeengt. Man erhält 124 g weißes Kristallisat, das zu 97,5 % rein ist. Eine Destillation (Sdp. 140°C/0,1 mbar) ergibt das reine Produkt; Fp. 77,8 bis 78,6°C; $\alpha_D^{20}$ = -66,3° (10 %ig in Ethanol).

Beispiel 7

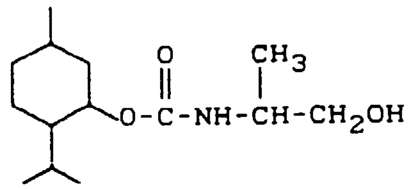

Verfahren analog Beispiel 6 aus 100 g (0,46 mol) Chlorameisensäurementhylester und 82,6 g (1,1 mol) 2-Aminopropanol-1. Ausbeute: 96,6 % der Theorie, bezogen auf Chlorameisensäurementhylester. Fp. 72 - 72,5°C; $\alpha_D^{20}$ = -63,3° (10 %ig in Ethanol).

Beispiel 8

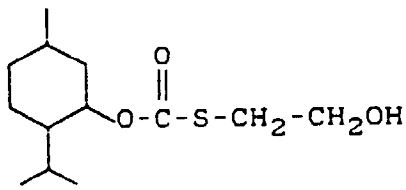

Verfahren analog Beispiel 1 aus 100 g (0,46 mol) Chlorameisensäurementhylester und 43 g (0,55 mol) Monothioethylenglykol. Durch Destillation (Sdp. 130,4 - 133,7°C/ 0,3 mbar) erhält man eine Mischung (49 % d. Theorie, bezogen auf Chlorameisensäurementhylester) aus 2/3 der gewünschten Verbindung und 1/3 der Verbindung

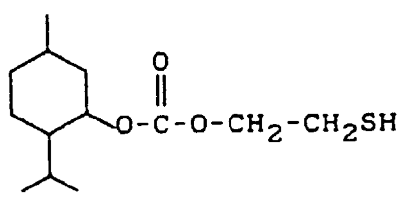

$D_{25}^{25}$ = 1,0483; $n_D^{25}$ = 1,4870; $\alpha_D^{20}$ = -62,5° (10 %ig in Ethanol).

Beispiel 9

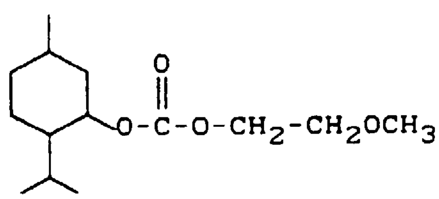

Herstellung analog Beispiel 1 aus 100 g (0,46 mol) Chlorameisensäurementhylester und 42 g (0,55 mol) 2-Methoxyethanol; Isolierung durch Destillation (Sdp. 125 bis 130°C/0,2 mbar) ohne nachfolgende Hexankristallisation. Man erhält eine Ausbeute von 93,4 % d. Theorie, bezogen auf Chlorameisensäurementhylester.
$D_{25}^{25}$ = 0,9949; $n_D^{25}$ = 1,4499; $\alpha_D^{20}$ = -62,5° (10 %ig in Ethanol).

Beispiel 10

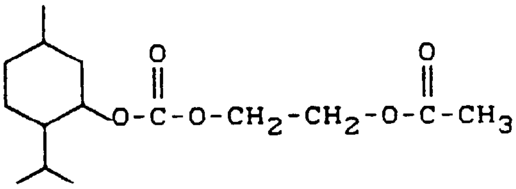

Verfahren analog Beispiel 1 aus 100 g (0,46 mol) Chlorameisensäurementhylester und 57,2 g (0,55 mol) Ethylenglykolmonoacetat. glykolmonoacetat. Durch Destillation (Sdp. 120-120,5°C/ 0,2 mbar) erhält man 80,1 g der gewünchten Verbindung. $D_{25}^{25}$ = 1,0411; $n_D^{25}$ = 1,4530; $\alpha_D^{20}$ = -56,2° (10 %ig in Ethanol).

Beispiel 11

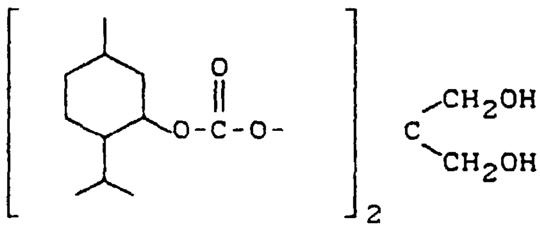

Herstellung analog Beispiel 1 aus 109 g (0,5 mol) ChlorameisensäuremenLhylesLer und 136 g (1 mol) Pentaerythrit in Gegenwart von 79,9 g (1,01 mol) Pyridin; man erhält ein Gemisch aus Mono-, Di- und Tricarbonat. Das Dicarbonat besitzt die stärkste Kühlwirkung. Das Gemisch besitzt folgende Eigenschaften:
$n_D^{20}$ = 1,4739; $\alpha_D^{20}$ = -50° (10 %ig in Ethanol).

Beispiel 12

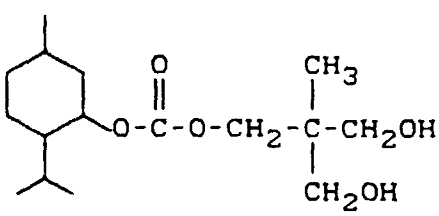

Verfahren analog Beispiel 11 aus 109 g (0,5 mol) Chlorameisensäurementhylester und 120 g (1 mol) 2,2,2-Trimethylolethan in Gegenwart von 79,9 g (1,01 mol) Pyridin; durch Destillation erhält man 73 % d. Theorie, bezogen auf Chlorameisensäurementhylester, des gewünschten Produkts.
$n_D^{20}$ = 1,4742; $\alpha_D^{20}$ = -56,8° (10 %ig in Ethanol).

Beispiel 13

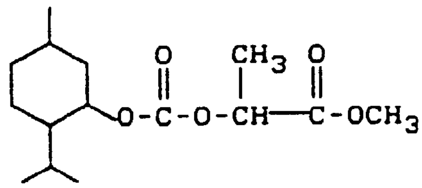

Man legt 65 g (0,55 mol) Milchsäureethylester in 400 ml MTBE vor, gibt 43,5 g (0,55 mol) Pyridin zu und läßt 100 g (0,46 mol) Chlorameisensäurementhylester innerhalb von 2,5 h zutropfen und weitere 2 h bei Raumtemperatur nachreagieren.

Entstandenes Pyridinium-hydrochlorid wird in 100 ml Wasser gelöst. Die organische Phase wird je einmal mit 10 %iger Salzsäure, konzentrierter wäßriger Natriumhydrogencarbonat-Lösung und mit Wasser gewaschen. Durch fraktionierte Destillation (Sdp. 130,4 - 135,7°C/1,5 mbar) erhält man 84,4 % d. Theorie, bezogen auf Chlorameisensäurementhylester, des gewünschten Produkts. $D^{25}_{25}$ = 1,0167; $n^{20}_{D}$ = 1,4480; $\alpha^{20}_{D}$ = -71,5° (10 %ig in Ethanol).

Beispiel 14

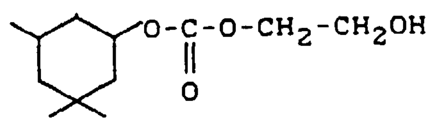

600 ml Hexan werden mit 119 g (0,4 mol) Triphosgen vorgelegt; 142 g (1 mol) 3,3,5-Trimethylcyclohexanol und 79 g (1 mol) Pyridin werden in 200 ml Hexan gelöst und bei Raumtemperatur in etwa 2 h zugetropft. Nach 12 Stunden Nachreaktionszeit wird ein Gemisch aus 62 g (1 mol) Ethylenglykol, 79 g (1 mol) Pyridin und 100 ml Hexan zugetropft, gefolgt von erneut 12 Stunden Nachreaktion. Der Ansatz wird zur Lösung des ausgefallenen Pyridiniumhydrochlorids mit 250 ml Wasser gerührt, die Phasen getrennt, die organische Phase 1x mit 100 ml 10 %iger Salzsäure, 1x mit 100 ml 10 %iger wäßriger $NaHCO_3$-Lösung und 2x mit 100 ml Wasser gewaschen.

Die fraktionierte Destillation ergibt das Produkt im Siedebereich von 112-113°C bei 0,5 mbar.
$D^{25}_{25}$ = 1,0463; $n^{20}_{D}$ = 1,4578
Ausbeute: 46,3 % der Theorie.

**Patentansprüche**

1. Verfahren zur Erzeugung eines physiologischen Kühleffekt auf der Haut oder Schleimhaut durch Aufbringen mindestens einer Verbindung der Formel

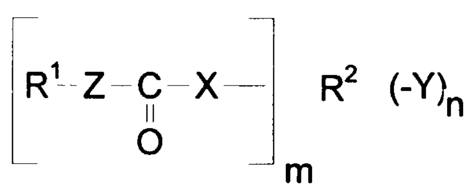

(I)

worin

| | |
|---|---|
| $R^1$ | $C_4$-$C_{20}$-Alkyl, $C_5$-$C_{20}$-Cycloalkyl oder -Heterocycloalkyl oder $C_5$-$C_{20}$-Alkoxy, $C_6$-$C_{12}$-Aryl, $C_5$-$C_{10}$-Heteroaryl oder $C_7$-$C_{11}$-Aralkyl, |
| $R^2$ | einen m + w · n -wertigen aliphatischen $C_1$-$C_8$-Rest, cycloaliphatischen oder heterocycloaliphatischen $C_3$-$C_{15}$-Rest, araliphatischen $C_7$-$C_{20}$-Rest, einen Alkoxy-oder Acyloxy-haltigen aliphatischen $C_3$-$C_{15}$- |

Rest,

Z und X unabhängig voneinander -O-, -S- oder -NH-,

Y Hydroxy, $C_1$-$C_{10}$-Alkoxy, $C_2$-$C_6$-Acyloxy, Amino, Mercapto oder -O-$R^3$-O,

$R^3$ $C_1$-$C_6$-Alkyfen,

w die Wertigkeit des Restes Y und

m und n unabhängig voneinander ganze Zahlen von 1 bis 8 bedeuten mit der Maßgabe, daß die Summe m + n maximal 12 ist.

**2.** Verfahren nach Anspruch 1, wobei in Formel I

$R^1$ $C_1$-$C_3$-substituiertes Cyclohexyl,

$R^2$ einen aliphatischen $C_1$-$C_8$-Rest oder einen Alkoxy- oder Acyloxy-enthaltenden aliphatischen $C_3$-$C_{15}$-Rest,

Z, X jeweils Sauerstoff und

Y Hydroxyl, $C_1$-$C_{10}$-Alkoxy oder $C_2$-$C_6$-Acyloxy bedeuten.

**3.** Verfahren nach Anspruch 1, wobei die Verbindung I aus der Reihe

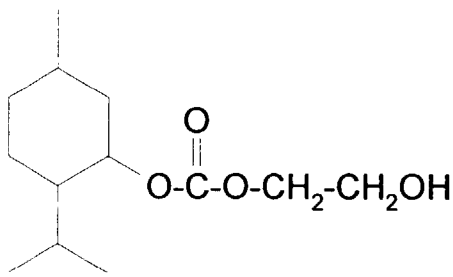

(II)

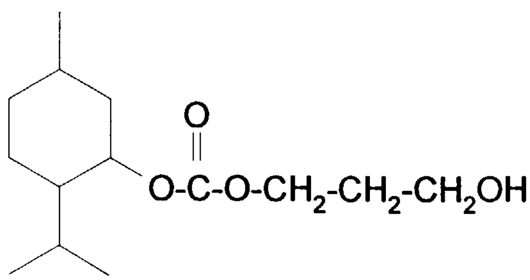

(III)

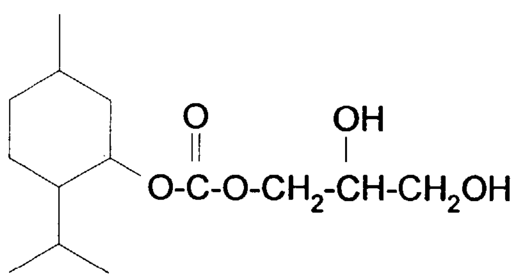

(IV)

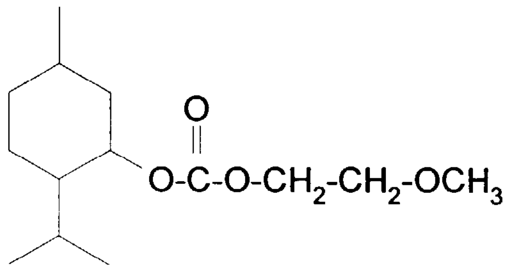

(V)

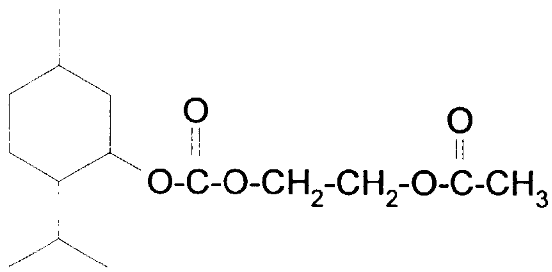

(VI)

ausgewählt ist.

**4.** Verbindungen der Formel I nach Anspruch 1 mit der Maßgabe, daß nicht gleichzeitig

| | |
|---|---|
| $R^1$ | Menthyl, |
| Z | Sauerstoff, |
| X | Sauerstoff, |
| $R^2(Y)_n$ | den Rest einer Polyhydroxylverbindung aus der Reihe Monosaccharide, Disaccharide, Trisaccharide, Polysaccharide, Glykole |

bedeuten.

**5.** Verbindungen nach Anspruch 4 der Formeln II, V und VI gemäß Anspruch 3.

**Claims**

**1.** Process for generating a physiological cooling effect on the skin or mucous membrane by application of at least one compound of the formula

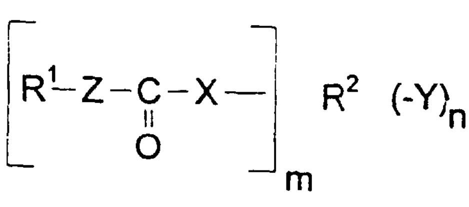

(I)

wherein

| | |
|---|---|
| $R^1$ | denotes $C_4$-$C_{20}$-alkyl, $C_5$-$C_{20}$-cycloalkyl or -heterocycloalkyl or $C_5$-$C_{20}$-alkoxy, $C_6$-$C_{12}$-aryl, $C_5$-$C_{10}$-heteroaryl or $C_7$-$C_{11}$-aralkyl, |
| $R^2$ | denotes an m + w · n-valent aliphatic $C_1$-$C_8$ radical, a cycloaliphatic or heterocycloaliphatic $C_3$-$C_{15}$ radical, an araliphatic $C_7$-$C_{20}$ radical, an alkoxy- or acyloxy-containing aliphatic $C_3$-$C_{15}$ radical, |
| Z and X | independently of one another denote -O-, -S- or -NH-, |

Y denotes hydroxyl, $C_1$-$C_{10}$-alkoxy, $C_2$-$C_6$-acyloxy, amino, mercapto or -O-$R^3$-O-,
$R^3$ denotes $C_1$-$C_6$-alkylene,
w denotes the valency of the radical Y and
m and n independently of one another denote integers from 1 to 8, with the proviso that the sum of m + n is not more than 12.

**2.** Process according to Claim 1, wherein, in formula I,

$R^1$ denotes $C_1$-$C_3$-substituted cyclohexyl,
$R^2$ denotes an aliphatic $C_1$-$C_8$-radical or an alkoxy-or acyloxy-containing aliphatic $C_3$-$C_{15}$-radical,
Z and X in each case denote oxygen and
Y denotes hydroxyl, $C_1$-$C_{10}$-alkoxy or $C_2$-$C_6$-acyloxy.

**3.** Process according to Claim 1, wherein the compound I is chosen from the series consisting of

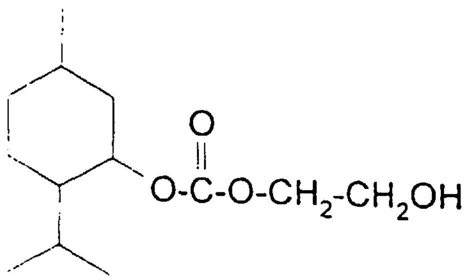

(II)

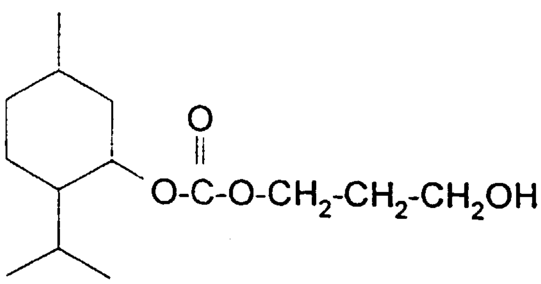

(III)

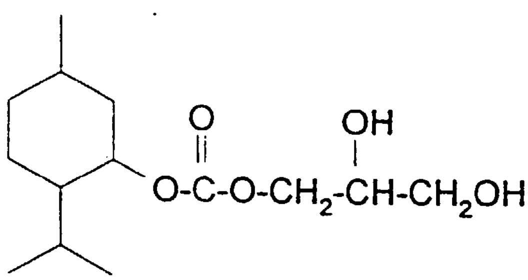

(IV)

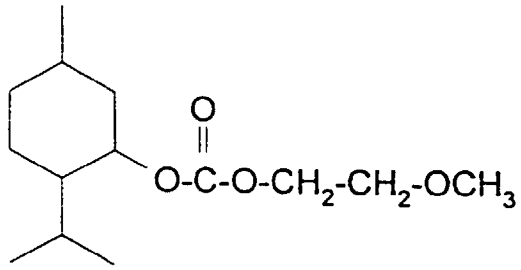

(V)

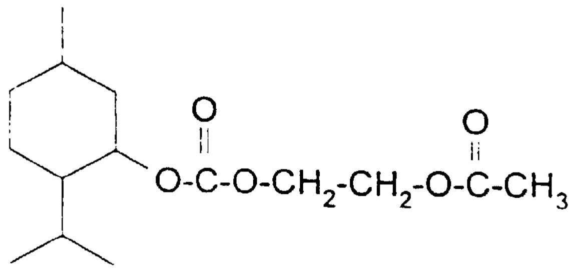

(VI)

**4.** Compounds of the formula I according to Claim 1, with the proviso that, simultaneously,

| | |
|---|---|
| $R^1$ | does not denote menthyl, |
| Z | does not denote oxygen, |
| X | does not denote oxygen, |
| $R^2(Y)_n$ | does not denote the radical of a polyhydroxy compound from the series consisting of monosaccharides, disaccharides, trisaccharides, pol ysaccharides and glycols. |

**5.** Compounds according to Claim 4 of the formulae II, V and VI according to Claim 3.

## Revendications

**1.** Procédé pour produire un effet de rafraîchissement physiologique sur la peau ou sur la muqueuse par application d'au moins un composé répondant à la formule

$$\left[R^1-Z-\underset{\underset{O}{\|}}{C}-X-\right]_m R^2\ (-Y)_n \qquad (I)$$

dans laquelle

| | |
|---|---|
| $R^1$ | représente un groupe alkyle en $C_4$-$C_{20}$, un groupe cycloalkyle ou un groupe hétérocycloalkyle en $C_5$-$C_{20}$ ou encore un groupe alcoxy en $C_5$-$C_{20}$, un groupe aryle en $C_6$-$C_{12}$, un groupe hétéroaryle en $C_5$-$C_{10}$ ou un groupe aralkyle en $C_7$-$C_{11}$, |
| $R^2$ | représente un radical aliphatique en $C_1$-$C_8$ à valence m + w · n, un radical cycloaliphatique ou hétérocycloaliphatique en $C_3$-$C_{15}$, un radical araliphatique en $C_7$-$C_{20}$, un radical aliphatique en $C_3$-$C_{15}$ contenant un groupe alcoxy ou un groupe acyloxy, |
| Z et X | représentent, indépendamment l'un de l'autre, -O-, -S- ou -NH-, |
| Y | représente un groupe hydroxyle, un groupe alcoxy en $C_1$-$C_{10}$, un groupe acyl(en $C_2$-$C_6$)oxy, un groupe amino, un groupe mercapto ou -O-$R^3$-O, |
| $R^3$ | représente un groupe alkylène en $C_1$-$C_6$, |

w représente la valeur du radical Y et

m et n représentent, indépendamment l'un de l'autre, des nombres entiers de 1 à 8, avec cette mesure que la somme m + n est égale au maximum à 12.

**2.** Procédé selon la revendication 1, dans lequel dans la formule I

$R^1$ représente un groupe cyclohexyle substitué en $C_1$-$C_3$,

$R^2$ représente un radical aliphatique en $C_1$-$C_8$ ou un radical aliphatique en $C_3$-$C_{15}$ contenant un groupe alcoxy ou un groupe acyloxy,

Z,X représentent respectivement un atome d'oxygène et Y représente un groupe hydroxyle, un groupe alcoxy en $C_1$-$C_{10}$ ou un groupe acyl(en $C_2$-$C_6$)oxy.

**3.** Procédé selon la revendication 1, dans lequel le composé I est choisi parmi la série comprenant

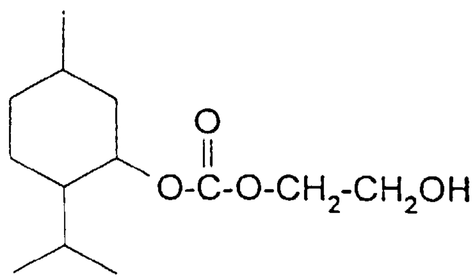

(II)

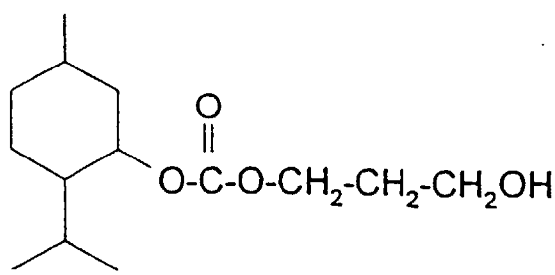

(III)

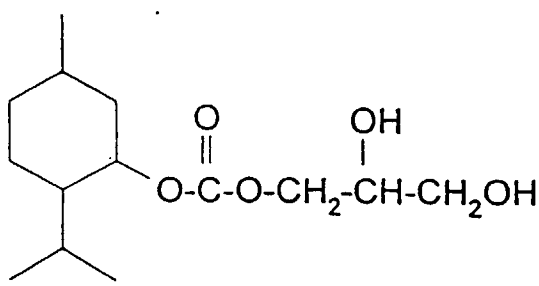

(IV)

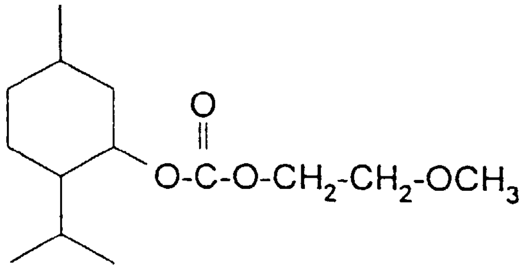

(V)

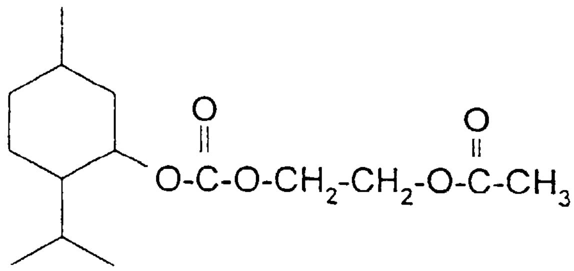

(VI)

**4.** Composés de formule I selon la revendication 1, avec cette mesure que les radicaux ci-après ne représentent pas simultanément

| | |
|---|---|
| $R^1$ | un groupe menthyle, |
| Z | un atome d'oxygène, |
| X | un atome d'oxygène, |
| $R^2(Y)_n$ | le radical d'un composé polyhydroxylé choisi parmi la série comprenant des monosaccharides, des disaccharides, des trisaccharides, des polysaccharides, des glycols. |

**5.** Composés selon la revendication 4, répondant aux formules II, V et VI selon la revendication 3.